Europäisches Patentamt

(19) European Patent Office

Office européen des brevets

(11) Numéro de publication: **0 090 688 B1**

(12) **FASCICULE DE BREVET EUROPEEN**

(45) Date de publication du fascicule du brevet:
24.05.89

(21) Numéro de dépôt: **83400444.2**

(22) Date de dépôt: **04.03.83**

(51) Int. Cl.⁴: **A 61 L 9/01, A 61 L 9/04**

(54) Procédé pour réduire la désorption par l'eau d'une composition active portée par un support.

(30) Priorité: **04.03.82 FR 8203599**

(43) Date de publication de la demande:
**05.10.83 Bulletin 83/40**

(45) Mention de la délivrance du brevet:
**24.05.89 Bulletin 89/21**

(84) Etats contractants désignés:
**AT BE CH DE FR GB IT LI LU NL SE**

(56) Documents cités:
**FR-A- 1 590 898**
**FR-A- 1 603 822**

**The Merck Index (1976), pp. 1251,1252,1258,1259**
**Kirk-Othmer "Encyclopedia of Chemical Technology**
**(19), pp. 340,341,346,348**

**Le dossier contient des informations techniques**
**présentées postérieurement au dépôt de la demande et**
**ne figurant pas dans le présent fascicule.**

(73) Titulaire: **Reckitt & Colman S.A., 15, rue Ampère,**
**F-91301 Massy Cédex (FR)**

(72) Inventeur: **Weiss, Richard, 32 rue d'Aligre,**
**F-28000 Chartres (FR)**
Inventeur: **Delagneau, Hubert, 14, rue des Tilleuls**
**Baileau l'Eveque, F-28300 Mainvilliers (FR)**

(74) Mandataire: **Rinuy, Santarelli et al, 14, avenue de la**
**Grande Armée, F-75017 Paris (FR)**

## Description

L'invention concerne un procédé pour éviter la désorption par l'eau d'une substance chimique active liquide portée par un support absorbant.

On connait de nombreux cas où une ou plusieurs substances chimiques sont appliquées sur des substrats naturels ou synthétiques en vue notamment de leur conversation et/ou de leur protection contre des agressions extérieures d'ordre chimique, biologique ou autre. On citera, par exemple, la protection des plantes, des pierres, du bois, du cuir, des organes vivants, l'hygiène corporelle, etc.

On connait également d'autres nombreux cas où une ou plusieurs substances chimiques sont appliquées sur des substrats naturels ou synthétiques en vue notamment d'un échange avec l'environnement, le milieu ambiant ou un autre support. On citera par exemple le cas de la libération progressive d'un parfum ou d'une substance active pour la désodorisation d'ambiances, la désinfection d'atmosphères, la lutte contre les insectes (brevet français N° 1 590 898).

Or il se trouve que dans plusieurs applications, le substrat porteur de la substance active est exposé en permanence, périodiquement ou encore accidentellement, à l'action de l'eau.

Dans de nombreux cas, cette eau provoque la désorption progressive et souvent rapide des principes actifs du substrat, d'où une perte importante de matière active et une diminution dans le temps de l'efficacité du système. Pour éviter ou du moins réduire les conséquences de ce phénomène, on fait appel à des procédés ou à des moyens qui sont, pour l'essentiel, des procédés ou des moyens d'hydrofugation et d'imperméabilisation. Ces moyens et procédés peuvent être classés en trois grandes catégories:

1. Hydrofugation préalable du support avant application du ou des principes actifs, l'hydrofugeant étant appliqué directement ou en solution dans un solvant ou encore en émulsion dans l'eau.

2. Application du ou des principes actifs sur le substrat, suivie de l'hydrofugation, l'hydrofugeant étant appliqué directement ou en solution dans un solvant, ou encore en émulsion dans l'eau.

3. Incorporation d'un hydrofugeant dans la ou les substances actives sous la forme d'une solution ou d'une émulsion, puis application de cette composition sur le substrat.

Or, ces procédés présentent toutefois dans un grand nombre de cas des inconvénients majeurs:

1) D'une façon générale, et pour lutter contre les conséquences de la désorption par l'eau des principes actifs, on est obligé d'incorporer des quantités importantes d'hydrofugeant, ce qui limite d'autant les quantités de substance(s) active(s) à faire porter par le substrat;

2) Le procédé d'hydrofugation préalable du support par incorporation de l'hydrofugeant sous la forme d'une solution dans un solvant ou sous la forme d'une émulsion, nécessite toujours une opération d'élimination du véhiculant (opération qui ne peut qu'être coûteuse) avant application du ou des principes actifs. De plus, lorsque l'on a recours à une émulsion dans l'eau de l'hydrofugeant, l'émulsifiant utilisé reste en général sur le substrat et provoque une sensible diminution de la qualité de l'hydrofugation;

3) Lorsqu'on procède à l'hydrofugation après application du ou des principes actifs sur le substrat, on note les mêmes inconvénients que ceux signalés précédemment. De plus, lorsque le substrat est destiné à porter des compositions volatiles en vue de leur évaporation progressive dans l'atmosphère, les quantités d'hydrofugeant qu'on est obligé de mettre en œuvre sont telles qu'elles modifient dans de nombreux cas l'évaporation normale de ces compositions si elles ne la bloquent pas totalement;

4) Très souvent, les procédés d'hydrofugation connus n'arrêtent ni ne modifient le phénomène de désorption par l'eau du ou des principes actifs portés par les substrats;

5) Dans de nombreux cas, les procédés d'hydrofugation connus ne sont pas applicables, notamment quand on désire traiter des substrats tels que des tissus animaux, des tissus végétaux, des tissus humains ou encore des substrats ou matières naturelles dont on ne veut pas modifier l'état ou l'aspect originel.

Or la Demanderesse a trouvé d'une façon fortuite et surprenante qu'il était possible de réduire au minimum la désorption par l'eau d'un principe actif porté par un substrat en agissant d'une part, par des moyens connus en soi, sur les charges ioniques respectives des composants de l'ensemble de manière que la charge ionique finale du substrat soit opposée à celle de la composition du ou des principes actifs et, d'autre part, sur la balance lipophile/hydrophile (HLB) de la composition de principe actif pour avoir dans le système final une tension de surface du couple support-composition de principe actif inférieure à la tension de surface du couple support absorbant-eau.

On entend dans la présente description par support, toute substance poreuse capable d'absorber ou d'adsorber une substance liquide indépendamment de l'importance de la quantité absorbée ou adsorbée de cette substance.

Pour atteindre ce résultat, l'invention fournit un procédé simple et économique pour réduire au minimum la désorption par l'eau d'une composition chimique active portée par un substrat lorsque cet ensemble est mis au contact d'un milieu aqueux de façon permanente, périodique ou accidentelle, ce procédé étant caractérisé par le fait qu'il consiste à traiter le substrat ou le liquide d'imprégnation destiné à être absorbé par ce substrat, par un composé polaire de façon à induire une polarité inverse de celle du substrat et du liquide d'imprégnation, de manière à obtenir une balance hydrophile/lipophile (HLB) telle que la tension de surface du couple support-composé actif soit inférieure à la tension de surface du couple support-eau.

On notera que le procédé de l'invention s'applique de préférence à des substrats poreux.

Ainsi, la ou les substances polaires de traitement sont choisies en fonction de la nature ionique de chacun des constituants du système final à réaliser et de la balance lipophile/hydrophile de la substance ou composition chimique active.

La mise en œuvre du procédé selon l'invention consiste donc soit à traiter la substance ou la composition chimique active par au moins un agent compatible avec cette substance ou composition et qui sera choisi de façon à conférer à la composition chimique résultante une polarité inverse de celle du substrat, soit à traiter le substrat pour lui conférer une polarité inverse de celle de la composition chimique, la polarité de cette dernière pouvant elle-même être modifiée, comme indiqué ci-dessus, pour lui conférer la polarité voulue. En particulier, lorsque la substance ou composition chimique active présente elle-même une polarité propre, on peut choisir l'agent de traitement dans le seul but de modifier la polarité du substrat, lorsque sa ionicité est déjà opposée à celle de la composition chimique active. De même, lorsqu'on désire utiliser, pour une raison ou une autre, un agent de traitement ayant une polarité donnée et appliquer la composition contenant cet agent sur un support électriquement neutre ou de même polarité que celle dudit agent, on peut procéder à un traitement conférant à ce support une polarité inverse de celle de ladite composition.

Ainsi, le ou les traitements ci-dessus, par la ou les substances polaires, peuvent être effectués avant, pendant ou après le dépôt de la composition active sur le substrat.

Toutefois, la quantité de substance polaire devra être suffisante pour obtenir les deux conditions conjuguées (polarité et HLB) sans modifier sensiblement ni les quantités relatives des constituants du système final, ni les caractéristiques essentielles de ces constituants ni leur mode d'action, en particulier celui de la substance ou de la composition chimique active. De préférence, cette quantité sera relativement faible par rapport à la composition active.

De la sorte, on ne modifie ni la nature du ou des corps en présence, ni leurs concentrations ni les quantités des principes actifs susceptibles d'être absorbées par le substrat, ni les modalités d'action du ou des principes actifs portés par ledit substrat. Ces conditions respectées sont d'autant plus intéressantes dans le cas particulier des substances volatiles dont les caractéristiques d'évaporation sont mises à profit en vue du traitement d'un environnement. En effet, dans ce cas, ces caractéristiques d'évaporation ne doivent pas être modifiées ou contrariées comme cela pouvait être le cas en faisant appel, pour lutter contre la désorption par l'eau, à une hydrofugation du substrat avant ou après imprégnation de ce dernier par la substance ou composition active.

Le procédé de l'invention utilise une composition empêchant ou retardant la désorption par un milieu aqueux d'une composition chimique liquide active fixée sur un substrat, composition caractérisée par le fait qu'elle comprend un support absorbant imprégné de ladite composition active et une quantité relativement faible d'au moins un composé ionique polaire tel que les charges du support et de la composition chimique active soient opposées et que la tension de surface du couple support absorbant-substance chimique active soit inférieure à la tension de surface du couple support absorbant-eau.

Le composé ionique polaire est avantageusement choisi parmi les composés énumérés ci-dessus.

En particulier, la composition chimique active peut être un parfum, un insecticide.

L'application du procédé et de la composition pour sa mise en œuvre permets la réalisation de systèmes résistant à l'eau renfermant des compositions de substances actives et/ou les substrats traités dans ce but.

Comme exemples d'agents de traitement convenant dans le procédé selon l'invention, on citera les composés appartenant aux classes suivantes:

1) Dans le cas de supports à charge négative lorsque la substance imprégnante ne possède pas une polarité propre:

– les composés contenant au moins un hétéro-atome $p^+$, $O^+$, $S^+$ ou $N^+$ et au moins une longue chaîne alkyle d'au moins 8 atomes de carbone tels que les sels d'ammonium quaternaire, les sels d'imidazoline, les sels de phosphonium, les sels d'oxonium, les amines grasses et leurs dérivés.

2) Dans le cas des supports à charge positive lorsque la substance imprégnante ne possède pas une polarité propre:

– les composants contenant au moins un groupement à caractère anionique tel que carboxylique, sulfonique, phosphorique, sulfurique et au moins une longue chaîne alkyle d'au moins 8 atomes de carbone tels que les acides alkylphosphoniques, alkylsulfuriques, alkylsulfoniques, alkylcarboxyliques.

3) Dans le cas où la substance ou la composition imprégnante possède une polarité propre:

a) polarité propre négative, le support étant à charge positive:

– les composés mono- ou poly-fonctionnels tels que bases organiques, bases minérales;

– les composés à caractère au moins bi-fonctionnel présentant au moins un groupement à caractère ionique négatif et ayant de plus un caractère ionique positif supérieur à ceux du groupement ionique du support tels que polyamines mono-carboxyliques, peptides, sulfo-bétaïnes;

a') polarité propre négative, le support étant à charge négative:

– les composés bi- ou poly-fonctionnels comportant au moins deux groupements ioniques positifs tels que polyamines, polyammoniums quaternaires;

b) polarité propre positive, le support étant à charge positive:

– les composés comportant au moins deux groupements ioniques négatifs tels que les polyacides;

b') polarité propre positive, le support étant à charge négative:

– les composés mono- ou poly-fonctionnels tels que les acides minéraux et les acides organiques;

– les composés à caractère au moins bi-fonctionnel présentant au moins un groupement à caractère ionique positif ayant de plus un caractère ionique négatif supérieur à ceux des groupements ioniques du support tels que les polyacides monoamines, les amino-acides, les peptides, les sulfo-bétaïnes.

Le tableau suivant illustre le procédé selon l'invention:

| S | I | $A_S$ | $A_I$ |
|---|---|---|---|
| 0 | 0 | + | − |
|   | 0 | − | + |
|   | + | − |   |
|   | − | + |   |
| + | 0 |   | − |
|   | + | − |   |
|   |   |   | − |
|   |   | ↗ + |   |
|   | − |   | ↗ − |
|   |   | ↗ + | ↗ − |
| − | 0 |   | + |
|   | + | ↗ − |   |
|   |   |   | ↗ + |
|   |   | ↗ − | ↗ + |
|   | − | + |   |
|   |   |   | + |

Sur ce tableau, la colonne «S» donne la polarité du substrat, la colonne «I» celle de la substance ou composition imprégnante, la colonne «$A_S$» la polarité de l'agent de traitement du substrat et la colonne «$A_i$» celle de l'agent de traitement de la substance ou de la composition imprégnante; les flèches montrent l'augmentation de la polarité résultante.

L'intérêt et les avantages de l'invention ressortiront plus clairement de la description qui va suivre et des exemples expérimentaux donnés à titre illustratif et nullement limitatif. Ces exemples illustrent l'application de l'invention dans les cas suivants:

* Fixation d'une composition de substances actives ne possédant pas de caractère ionique propre:

– sur un support absorbant possédant un caractère ionique négatif

– sur un support absorbant possédant un caractère ionique positif

\* Fixation d'une composition de substances actives possédant un caractère ionique propre par modification de la ionicité du support absorbant

\* Fixation d'une composition de substances actives ne possédant pas de caractère ionique propre par un additif défini en modifiant la ionicité du support absorbant.

La méthode généralement utilisée est la suivante: Les compositions de substances actives à tester sont colorées le cas échéant à l'aide de colorants non solubles à l'eau, puis selon la présente invention, imprégnées sur le support absorbant, puis finalement immergées dans de l'eau maintenue à température ambiante.

La désorption de la composition de substances actives est appréciée visuellement dans le temps. On note par:

0: aucune désorption
1: désorption faible
2: désorption partielle
3: désorption quasi totale.

EXEMPLES

Exemple 1:

Fixation d'une composition de substances actives ne possédant pas de caractère ionique propre sur un support possédant un caractère ionique négatif:

– Matériel:
  – Support absorbant: A titre illustratif d'une substance absorbante on a choisi une plaquette de cellulose (disponible sur le marché sous l'appellation «Spartose OM-22»® de la Société La Rochette Cenpa)
  – Composition de substances actives: dodécane (disponible sur le marché sous l'appellation «Solis»® de la Société B.P.)
  – Additif: chlorure de distéaryl diméthyl ammonium quaternaire
– Résultats:
  – L'additif a été introduit dans la composition de substances actives à la concentration de 2% en poids
  – Les résultats figurent au tableau I ci-après:

Tableau I

| Conditions | Temps en mn | | | | |
| | 1 | 15 | 30 | 45 | 60 |
| --- | --- | --- | --- | --- | --- |
| Composition seule | 1 | 2,5 | 2,5 | 2,5 | 2,5 |
| Composition modifiée | 0 | 0 | 0 | 0 | 0 |

Exemple 2:

Fixation d'une composition de substances actives ne possédant pas de caractère ionique propre sur un support absorbant possédant un caractère ionique positif:

– Matériel:
  – Support absorbant: résine cationique référence «Ionenaustauscher III» de la Société Merk
  – Composition de substances actives: dodécane référence «Solis»® (B.P.)

– Additif: acide oléique
– Résultats:
  – Pour cet exemple, le support absorbant a été traité séparément par l'additif, de la façon suivante: Traitement pendant 30 minutes par une solution alcoolique à 2% en poids d'acide oléique, ou par de l'alcool pour l'essai témoin, filtration puis séchage à poids constant avant imprégnation par la composition de substances actives
  – La désorption de la composition de substances actives a été évaluée après 2 heures d'immersion.
  – Les résultats figurent au tableau II ci-après:

Tableau II

| Conditions | Desorption après 120 mn |
| --- | --- |
| Support non traité | 3 |
| Support traité | 0 |

Exemple 3:

Fixation d'une composition de substances actives possédant un caractère ionique propre par modification de la ionicité du support absorbant

– Matériel:
  – Support absorbant: plaquette de cellulose référence «Spartose OM-22»® de la Société La Rochette Cenpa
  – Composition de substances actives: salicylate de méthyle
– Modification de la ionicité du support: La ionicité négative propre du support est augmentée par un traitement par un acide inorganique fort. Le support est immergé 30 minutes dans une solution à 5% en poids d'acide chlorhydrique techniquement pur, rincé à l'eau déminéralisée courante 1 minute et séché à poids constant.
– Les résultats figurent au tableau III ci-après:

Tableau III

| Conditions | Temps en mn | | | | |
| | 1 | 15 | 30 | 45 | 60 |
| --- | --- | --- | --- | --- | --- |
| Support non traité | 1 | 1,5 | 2 | 2,5 | 2,5 |
| Support traité | 0 | 0,5 | 0,5 | 1 | 1 |

Exemple 4:

Fixation d'une composition de substances actives ne possédant pas de caractère ionique propre par un additif défini en modifiant la ionicité du support absorbant
– Matériel:
  – Support absorbant: plaquette de cellulose référence «Spartose OM-22»® (Société La Rochette Cenpa).
  – Composition de substances actives: composition parfumante type Pin, référence «Tanalis»® (Société Naarden)
  – Additif: acide oléique

- Modification de la ionicité du support: Le support est traité afin de lui donner un caractère ionique positif opposé à celui de l'additif choisi. Le support est traité 30 minutes par une solution d'acide chlorhydrique techniquement pur à 5% en poids, rincé à l'eau déminéralisée courante pendant 1 minute puis traité pendant 30 minutes par une solution d'éthylène diamine à 5% en poids, rincé à l'eau déminéralisée courante pendant 1 minute puis finalement séché à poids constant. L'additif a été introduit dans la composition de substances actives à la concentration de 2% en poids.
- Résultats:
  - les résultats figurent au table IV ci-après:

Tableau IV

| Conditions | Temps en mn | | | | |
|---|---|---|---|---|---|
| | 1 | 15 | 30 | 45 | 60 |
| Cellulose non traitée Témoin composition type pin | 0,5 | 2 | 2,5 | 3 | 3 |
| Composition type pin traitée avec l'acide oléique | 1 | | 2,5 | 3 | 3 | 3 |
| Cellulose traitée Témoin composition type pin | 1 | 2 | 2,5 | 3 | 3 |
| Composition type pin traitée avec l'acide oléique | 0 | 0,5 | 1 | 1,5 | 1,5 |

Conclusions:

Les résultats obtenus montrent clairement que la présente invention permet d'éviter ou de réduire au minimum le phénomène de désorption par l'eau de compositions de substances actives imprégnées sur des supports absorbants et donc ainsi d'éviter ou de freiner l'élimination desdites compositions de substances actives desdits supports lorsque ceux-ci sont mis au contact en permanence, périodiquement ou accidentellement d'eau ou de solutions aqueuses diluées.

Par ailleurs, pour démontrer clairement les intérêts techniques et économiques de la présente invention, la demanderesse a prouvé:

– qu'il est possible d'éviter la désorption par l'eau d'une composition insecticide imprégnée sur un support absorbant et donc de réaliser de nouveaux types de produits insecticides pouvant être, sans perdre leur efficacité, exposés à la pluie ou à l'humidité;

– qu'il est possible d'éviter la désorption par l'eau de compositions parfumantes imprégnées sur un support absorbant et donc de réaliser de nouveaux types de produits déodorants (ou réodorants) d'ambiance, insensibles à l'eau ou à l'humidité, pouvant, par exemple, sans perdre leur efficacité, être fixés sous le rebord des cuvettes de W.C.

Exemple 5:

Fixation d'une composition insecticide sur un support absorbant:

La fixation de la composition insecticide sur le support absorbant a été évaluée selon la méthode d'immersion décrite précédemment.

- Matériel:
  - Support absorbant: plaquette de cellulose référence «Spartose OM-22»® de la Société La Rochette Cenpa
  - Composition insecticide: composition pouvant être utilisée pour le traitement insecticide de plantes. Formule en partie en poids:

| | |
|---|---|
| Pyréthrine | 0,05 |
| Pipéronyl butoxide | 0,25 |
| Roténone | 0,025 |
| Chloropropylate | 0,05 |
| Dichlone | 0,1 |
| Dinocap | 0,05 |
| Lindane | 0,1 |
| Dichlorométhane | 26,7 |

- Additif: chlorure de distéaryl diméthyl ammonium quaternaire
- Résultats:
  - L'additif a été introduit dans la composition insecticide à la faible concentration de 5% en poids
  - Les résultats figurent au tableau V ci-après:

Tableau V

| Conditions | Temps en mn | | | | |
|---|---|---|---|---|---|
| | 1 | 15 | 30 | 45 | 60 |
| Témoin: composition seule | 1 | 2 | 2,5 | 2,5 | 2,5 |
| Composition modifiée | 0 | 0 | 0 | 0 | 0 |

- Conclusions:

Les résultats obtenus montrent clairement que la présente invention peut être avantageusement mise en œuvre lorsqu'on cherche à éviter la désorption par l'eau de compositions insecticides destinées à être appliquées sur des supports absorbants.

Exemple 6:

Réalisation d'un déodorant d'ambiance à fixer sous le rebord des cuvettes de WC

* Etude de la désorption par l'eau de compositions parfumantes imprégnées sur un support absorbant:

Le degré de fixation des compositions parfumantes sur les supports absorbants a été évalué selon la méthode d'immersion déjà décrite.

Des compositions parfumantes formulées complexes et des parfums chimiquement définis ont été utilisés pour cette étude.

Les compositions à tester sont colorées à l'aide d'un colorant liposoluble non soluble à l'eau, puis imprégnées sur le support absorbant, et enfin immergées dans de l'eau maintenue à température ambiante.

La désorption de la composition parfumante est appréciée visuellement dans le temps.

On note par:

0: aucune désorption
1: désorption faible
2: désorption partielle
3: désorption quasi-totale.

— Matériel:
— Support absorbant: Plaquette de cellulose à caractère ionique négatif Référence «Spartose OM-22»® (Société La Rochette Cenpa)
— Compositions parfumantes:
— Composition type Pin complexe: référence «Tanalis»® (Naarden)
— Composition type Florale complexe: référence «Boronia»® (société Givaudan)
— Acétate de bornyle
— Salicylate de méthyle
— Additifs: Composés comportant au moins un groupement ionique à caractère cationique
— Additif A: chlorure de distéaryl diméthyl ammonium quaternaire
— Additif B: éthyl sulfate d'éthyl 1 stéarylamido-éthyl 1 stéaryl 2 imidazoline
— Additif C: hydroxyéthyl 1 heptadécényl 2 imidazoline
— Additif D: N stéarylamine
— Additif E: chlorure de stéaryl triméthyl ammonium quaternaire

Les concentrations en additif utilisées sont exprimées en pour cent en poids par rapport à la composition parfumante.

— Résultats:

Tableau VI:

composition parfumante type Pin

| Additif | Concentration en Additif | Temps en mn | | | |
|---|---|---|---|---|---|
| | | 1 | 4 | 20 | 60 |
| Témoin composition type Pin | | 0,5 | 1 | 3 | 3 |
| A | 0,6 | 0 | 0,5 | 1 | 2 |
| A | 2 | 0 | 0 | 0 | 0 |
| C | 5 | 0 | 0 | 0,5 | 1 |
| D | 10 | 0 | 0 | 1 | 1 |
| E | 5 | 0,5 | 1 | 1 | 1 |

Tableau VII:

composition parfumante type Florale

| Additif | Concentration en Additif | Temps en mn | | | |
|---|---|---|---|---|---|
| | | 1 | 4 | 20 | 60 |
| Témoin composition type Florale | | 1 | 3 | 3 | 3 |
| A | 2 | 0 | 0 | 0 | 0 |
| B | 2 | 0 | 0 | 0,5 | 1 |
| C | 2 | 0 | 1 | 1,5 | 2 |

Tableau VIII:

acétate de bornyle

| Additif | Concentration en Additif | Temps en mn | | | |
|---|---|---|---|---|---|
| | | 1 | 15 | 30 | 60 |
| Témoin acétate de bornyle | | 2 | 2,5 | 2,5 | 3 |
| A | 2 | 0 | 0 | 0 | 0 |
| B | 2 | 0 | 0 | 0 | 0 |
| C | 2 | 0,5 | 1 | 2,5 | 2,5 |

Tableau IX:

salicylate de méthyle

| Additif | Concentration en Additif | Temps en mn | | | |
|---|---|---|---|---|---|
| | | 1 | 15 | 30 | 60 |
| Témoin salicylate de méthyle | | 1,5 | 1,5 | 2 | 2,5 |
| B | 2 | 0,5 | 1 | 1,5 | 1,5 |
| C | 10 | 0 | 0 | 0 | 0 |

— Conclusions:

Ces résultats montrent que les systèmes faisant l'objet de la présente invention limitent, voire évitent quasi totalement la désorption par l'eau des compositions parfumantes imprégnées sur un support absorbant et qu'il est donc possible de les placer sous les rebords d'une cuvette de WC sans que les compositions soient extraites par l'eau au moment de l'actionnement de la chasse d'eau.

\* Etude de l'évaporation des compositions parfumantes:

a) Lorsque celles-ci sont incorporées dans des blocs détergents parfumants pour cuvettes de WC connus, sans paradichlorobenzène et avec paradichlorobenzène, (P.D.C.B.)

b) Lorsque ces mêmes compositions sont fixées sur un support absorbant

c) Lorsque ce même support absorbant est imprégné de compositions parfumantes et d'additifs selon la présente invention.

La méthode utilisée est la suivante:

— On suit par pesée l'évaporation des compositions parfumantes incorporées dans les blocs ou imprégnées sur des supports poreux, en quantité et surface d'évaporation équivalentes, dans des conditions constantes de mouvement d'air, de température et d'hygrométrie (20 °C, 25% H.R.). Dans les blocs qui contiennent du paradichlorobenzène, la perte de poids de la composition parfumante est déterminée par analyse chromatographique en phase gazeuse.

— On trace les courbes d'évaporation correspondant à la fonction:

$P = f(t)$ où — P est la perte de poids cumulée de la composition parfumante
— t est le temps d'évaporation

— Puis on établit les tableaux comparatifs:
— des valeurs de P en fonction de t

— des valeurs $\frac{dP}{dt}$ en fonction de t, déterminées graphiquement lorsque dt→0

— Les valeurs obtenues mesurent directement l'effet parfumant potentiel des compositions parfumantes

— Lorsque les compositions parfumantes sont incorporées dans des blocs nettoyants, la composition des blocs est la suivante (parties en poids):

|  | A | B |
|---|---|---|
| Dodécyl benzène sulfonate de sodium | 50 | 50 |
| Sulfate de soude | 8,5 | 8,5 |
| Cire de polyéthylène | 25 | 30 |
| Paradichlorobenzène | 5 | — |
| Silice précipitée | 5 | 5 |
| Composition parfumante | 6,5 | 6,5 |
| Colorants | traces | traces |

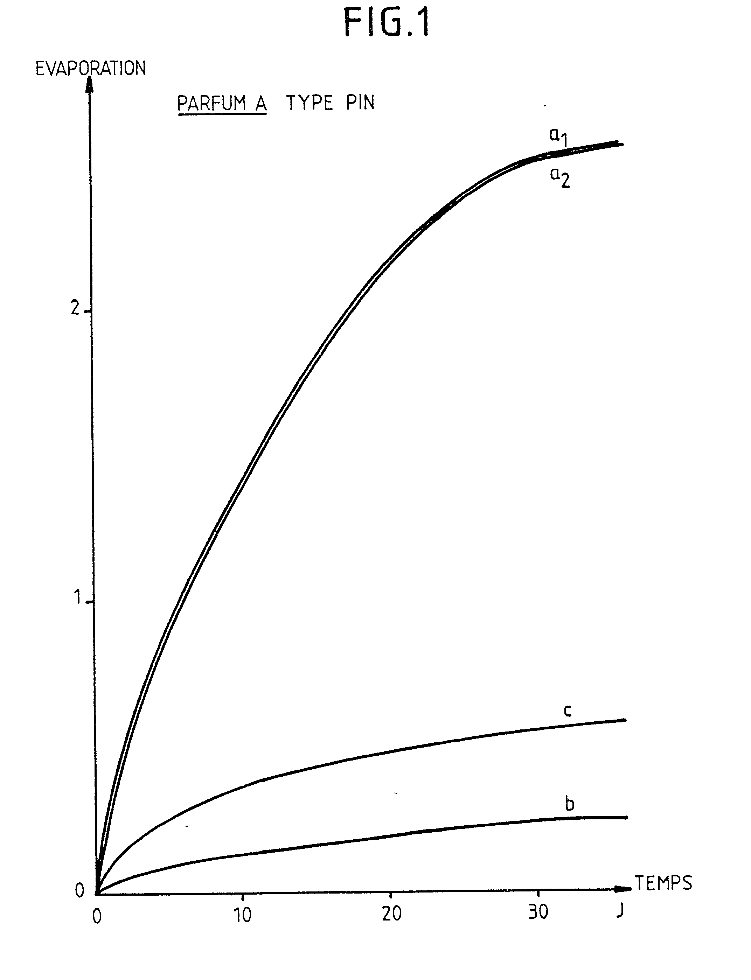

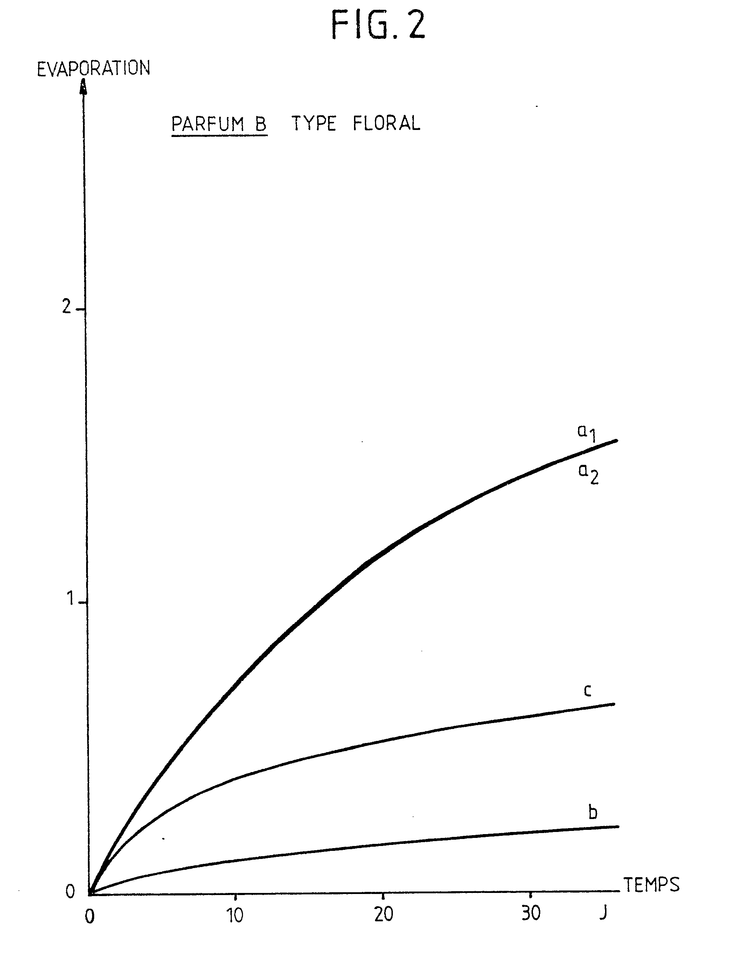

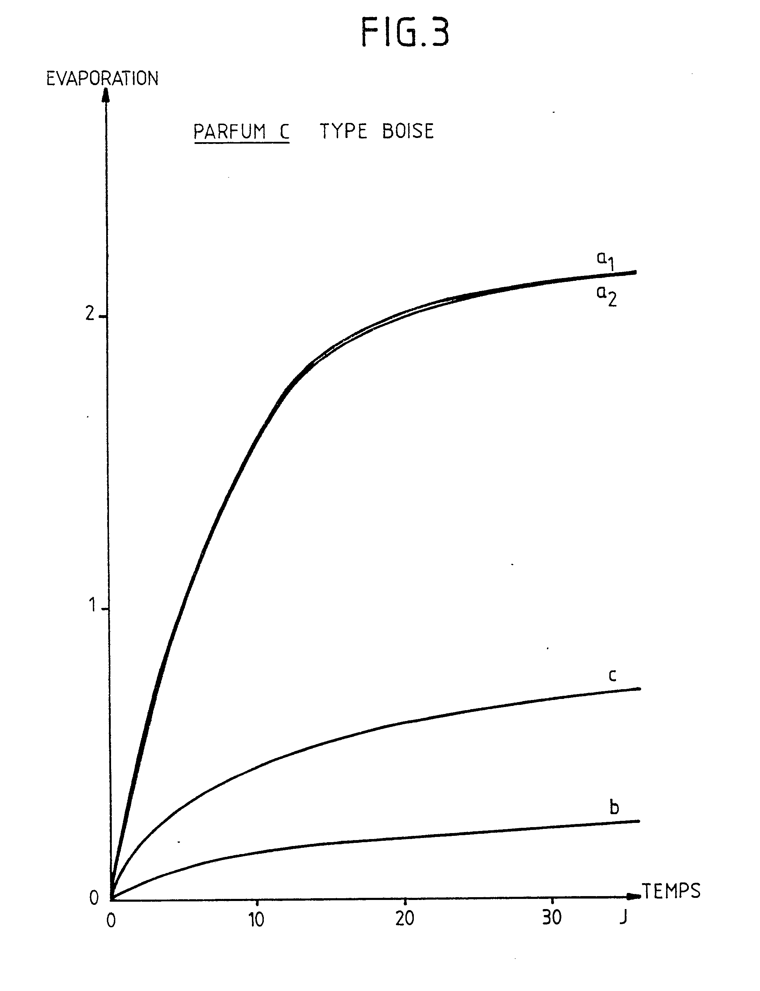

— Résultats:
— Les résultats obtenus sont regroupés dans les courbes d'évaporation des figures 1, 2 et 3 annexées et dans les tableaux suivants où:

$a_1$ est la composition parfumante imprégnée sur un support absorbant selon l'invention

$a_2$ est la composition parfumante imprégnée sur un support absorbant

b est la composition parfumante introduite dans un bloc détergent sans paradichlorobenzène

c est la composition parfumante introduite dans un bloc détergent contenant du paradichlorobenzène

Tableau X:

parfum A type Pin poids 2,6 g

| Temps d'évaporation | | P = f (t) | $\frac{\Delta P}{\Delta t}$ |
|---|---|---|---|
| (jours) | | g | g/jours |
| 5 | $a_1 = a_2$ | 0,92 | 0,12 |
|  | b | 0,09 | 0,01 |
|  | c | 0,28 | 0,03 |
| 10 | $a_1 = a_2$ | 1,4 | 0,12 |
|  | b | 0,12 | 0,01 |
|  | c | 0,35 | 0,03 |
| 15 | $a_1 = a_2$ | 1,84 | 0,10 |
|  | b | 0,17 | 0,01 |
|  | c | 0,43 | 0,015 |
| 20 | $a_1 = a_2$ | 2,16 | 0,07 |
|  | b | 0,2 | 0,005 |
|  | c | 0,47 | 0,01 |
| 30 | $a_1 = a_2$ | 2,5 | 0,02 |
|  | b | 0,2 | 0,05 |
|  | c | 0,56 | 0,007 |

Tableau XI:

parfum B type Floral Poids 2,6 g

| Temps d'évaporation | | P = f (t) | $\frac{\Delta P}{\Delta t}$ |
|---|---|---|---|
| (jours) | | g | g/jours |
| 5 | $a_1 = a_2$ | 0,42 | 0,08 |
|  | b | 0,07 | 0,015 |
|  | c | 0,3 | 0,03 |
| 10 | $a_1 = a_2$ | 0,74 | 0,06 |
|  | b | 0,12 | 0,01 |
|  | c | 0,4 | 0,03 |
| 15 | $a_1 = a_2$ | 0,96 | 0,05 |
|  | b | 0,16 | 0,01 |
|  | c | 0,47 | 0,02 |
| 20 | $a_1 = a_2$ | 1,16 | 0,03 |
|  | b | 0,18 | 0,005 |
|  | c | 0,52 | 0,02 |
| 30 | $a_1 = a_2$ | 1,45 | 0,025 |
|  | b | 0,2 | 0,0025 |
|  | c | 0,6 | 0,1 |

Tableau XII:

parfum C type Boisé poids 2,6 g

| Temps d'évaporation | | P = f (t) | $\frac{\Delta P}{\Delta t}$ |
|---|---|---|---|
| (jours) | | g | g/jours |
| 5 | $a_1 = a_2$ | 1 | 0,13 |
|  | b | 0,1 | 0,02 |
|  | c | 0,35 | 0,04 |
| 10 | $a_1 = a_2$ | 1,55 | 0,08 |
|  | b | 0,16 | 0,01 |
|  | c | 0,46 | 0,03 |
| 15 | $a_1 = a_2$ | 1,88 | 0,05 |
|  | b | 0,19 | 0,007 |
|  | c | 0,55 | 0,015 |
| 20 | $a_1 = a_2$ | 2,0 | 0,02 |
|  | b | 0,22 | 0,003 |
|  | c | 0,62 | 0,01 |
| 30 | $a_1 = a_2$ | 2,1 | 0,01 |
|  | b | 0,24 | 0,002 |
|  | c | 0,7 | 0,005 |

— Conclusions:
— Les résultats montrent clairement la supériorité de l'effet parfumant pouvant être obtenu à l'aide de la présente invention et l'intérêt économique qui en découle.

* Evaluation organoleptique des compositions parfumantes placées dans des conditions normales d'utilisation:

a) lorsque celles-ci sont incorporées dans des blocs détergents parfumants à base de paradichlorobenzène

b) lorsque celles-ci sont déposées sur un support absorbant

c) lorsque celles-ci sont fixées sur un support absorbant selon la présente invention.

Le pouvoir parfumant des compositions parfumantes est évalué par un «panel» approprié, 2 à 2, en parallèle, dans des cabines d'olfaction, avant et après mise en place dans des cuvettes de W.C. dont les chasses sont actionnées automatiquement selon la grille d'évaluation suivante:

1 Pas d'action parfumante
2 Action parfumante faible
3 Action parfumante correcte
4 Bonne action parfumante
5 Forte action parfumante

— Résultats:

a) Comparaison du pouvoir parfumant de plaquettes de cellulose identiques imprégnées d'une composition parfumante et imprégnées de la même composition parfumante selon la présente invention

Notes obtenues (moyennes)

| Nombre de chasses | Départ | Après 450 chasses (nombre de chasses obtenu en 6 jours) |
|---|---|---|
| Imprégnation classique | 3,4 | 2,7 |
| Imprégnation selon l'invention | 3,5 | 3,8 |

b) Comparaison du pouvoir parfumant d'une composition parfumante fixée selon la présente invention sur une plaquette de cellulose et introduite dans un bloc détergent à base de paradichlorobenzène, à poids et surface d'évaporation identiques

Notes obtenues (moyennes)

| Nombre de chasses | Départ | Après 150 chasses (nombre de chasses obtenu en 2 jours) |
|---|---|---|
| Cellulose imprégnée selon l'invention | 3,75 | 3,6 |
| Bloc détergent parfumant | 2,9 | 2,8 |

— Conclusions:

Les résultats obtenus montrent clairement la supériorité de l'effet obtenu selon la présente invention par rapport aux procédés connus habituels.

Il résulte de ce qui précède que le procédé selon l'invention peut être utilement mis en œuvre d'une façon générale chaque fois que l'on désire s'opposer au phénomène de désorption par l'eau d'une composition quelconque déposée ou à déposer sur un substrat et donc, d'une façon particulière lorsqu'une composition active volatile est déposée ou est à déposer sur un substrat approprié en vue du traitement de l'environnement ou encore lorsque la composition active doit être maintenue au contact du substrat en vue de la protection de ce dernier ou de son traitement.

**Revendications**

1. Procédé pour réduire la désorption de liquides absorbés par un substrat pendant la mise en contact d'un tel ensemble avec un milieu aqueux, caractérisé par un traitement du substrat ou du liquide d'imprégnation destiné à être absorbé par un composé polaire de façon à induire une polarité inverse du substrat et du liquide d'imprégnation de manière à obtenir une balance hydrophile/lipophile (HLB) telle que la tension de surface du couple support-composition active soit inférieure à la tension de surface du couple supporteau.

2. Procédé selon la revendication 1, caractérisé par le fait que le substrat est un absorbant poreux.

3. Procédé selon la revendication 1 ou 2, caractérisé par le fait que, suivant les polarités initiales en présence et les polarités finales à obtenir, les composés ioniques d'addition sont choisis dans les classes suivants comprenant:

— les composés contenant au moins un hétéroatome $P^+$, $O^+$, $S^+$ ou $N^+$ et au moins une longue chaîne alkyle d'au moins 8 atomes de carbone tels que les sels d'ammonium quaternaire, les sels d'imidazoline, les sels de phosphonium, les sels d'oxonium, les amines grasses et leurs dérivés;

— les composés contenant au moins un groupement à caractère anionique tel que carboxylique, sulfonique, phosphorique, sulfurique et au moins une longue chaîne alkyle d'au moins 8 atomes de carbone tels que les acides alkylphosphoniques, alkylsulfuriques, alkylsulfoniques, alkylcarboxyliques;

— les composés mono- ou polyfonctionnels tels que bases organiques, bases minérales; les composés à caractère au moins bifonctionnel présentant au moins un groupement à caractère ionique négatif et ayant de plus un caractère ionique positif supérieur à ceux du groupement ionique du support tels que polyamines monocarboxyliques, peptides, sulfo-bétaïnes;

— les composés bi- ou polyfonctionnels comportant au moins deux groupements ioniques positifs tels que polyamines, polyammoniums quaternaires;

— les composés comportant au moins deux groupements ioniques négatifs tels que les polyacides;

— les composés mono- ou polyfonctionnels tels que les acides minéraux et les acides organiques;

— les composés à caractère au moins bifonctionnel présentant au moins un groupement à caractère ionique positif ayant de plus un caractère ionique négatif supérieur à ceux des groupements ioniques du support tels que les polyacides monoamines, les amino-acides, les peptides, les sulfobétaïnes.

4. Procédé selon l'une quelconque des revendications 1 à 3, caractérisé par le fait que la quantité de substance polaire est suffisante pour obtenir

les deux conditions conjuguées (polarité et HLB) sans modifier sensiblement ni les quantités relatives des constituants du système final, ni les caractéristiques essentielles de ces constituants ni leur mode d'action, en particulier celui de la composition chimique active.

5. Procédé selon l'une quelconque des revendications 1 à 3, caractérisé par le fait que la quantité de substance polaire est relativement faible par rapport à la composition active liquide.

6. Composition empêchant ou retardant la désorption par un milieu aqueux d'une composition chimique liquide active fixée sur un substrat, composition caractérisée par le fait qu'elle comprend un support absorbant imprégné de ladite composition active et une quantité relativement faible d'au moins un composé ionique polaire tel que les charges du support et de la composition chimique active soient opposées et que la tension de surface du couple support absorbant-substance chimique active soit inférieure à la tension de surface du couple support-absorbant-eau.

7. Composition selon la revendication 7, caractérisée par le fait que la composition chimique active est un insecticide.

8. Application du procédé selon l'une quelconque des revendications 1 à 6 à la réalisation de systèmes résistant à l'action de l'eau renfermant au moins une composition chimique active liquide.

9. Application du procédé selon l'une quelconque des revendications 1 à 6 à la réalisation de déodorants d'ambiance pour cuvettes de W.C. et d'insecticides.

**Claims**

1. A process for reducing the desorption of liquids absorbed on a substrate while such an assembly is in contact with an aqueous medium, characterized by treating the substrate or the impregnation liquid which is to be absorbed with a polar compound in a manner which causes the substrate and the impregnation liquid to have opposite polarities so as to obtain a hydrophilic/lipophilic balance (HLB) such that the surface tension of the substrate/active composition couple is lower than the surface tension of the substrate/water couple.

2. A process according to claim 1, characterized in that the substrate is a porous absorbent material.

3. A process according to claim 1 or 2, characterized in that, depending on the initial polarities present and the final polarities to be obtained, the ionic additive compounds are selected from the following groups comprising:
— compounds containing at least one heteroatom $P^+$, $O^+$, $S^+$, or $N^+$ and at least one long chain alkyl of at least 8 carbon atoms, such as the quaternary ammonium salts, the imidazoline salts, the phosphonium salts and the fatty amines and their derivatives;
— compounds containing at least one group of anionic character, such as the carboxylic, sulphonic, phosphoric and sulphuric groups, and at least one long chain alkyl of at least 8 carbon atoms, such as the alkylphosphonic, alkylsulphuric, alkylsulphonic and alkylcarboxylic acids;
— mono or polyfunctional compounds such as organic bases and mineral bases; compounds of an at least bifunctional character, having at least one group of negative ionic character and having in addition a positive ionic character greater than that of the ionic groups of the substrate, such as monocarboxylic polyamines, peptides and sulphobetaines;
— bi or polyfunctional compounds comprising at least two positive ionic groups such as polyamines and quaternary polyammoniums;
— compounds comprising at least two negative ionic groups such as polyacids;
— mono or polyfunctional compounds such as mineral acids and organic acids;
— compounds of an at least bifunctional character, having at least one group of positive ionic character and having in addition a negative ionic character greater than that of the ionic groups of the substrate, such as monoamine polyacids, aminoacids, peptides and sulphobetaines.

4. A process according to any one of claims 1 to 3, characterized in that the quantity of polar substance is sufficient for obtaining both conjugated conditions (polarity and HLB) without modifying substantially the relative quantities of the constituents of the final system or the essential characteristics of these constituents or their mode of action, in particular that of the active chemical composition.

5. A process according to any one of claims 1 to 3, characterized in that the quantity of polar substance is relatively small in relation to the active liquid composition.

6. A composition preventing or delaying desorption by an aqueous medium of an active liquid chemical composition fixed on a substrate, this composition being characterized in that it comprises an absorbant support impregnated with the said active composition and a relatively low quantity of at least one polar ionic compound such that the support and the active chemical composition have opposite charges, and in that the surface tension of the absorbant support/active chemical substance couple is lower than the surface tension of the absorbant support/water couple.

7. A composition according to claim 7, characterized in that the active chemical composition is an insecticide.

8. Application of the process according to any one of claims 1 to 6 to the production of systems, resisting the action of water, containing at least one active liquid chemical composition.

9. Application of the process according to any one of claims 1 to 6 to the production of environment deodorants for toilet bowls and insecticides.

**Patentansprüche**

1. Verfahren zum Vermindern der Desorption von durch ein Substrat absorbierten Flüssigkeiten während des Kontaktes eines solchen Gebildes mit einem wässerigen Medium, gekennzeichnet durch eine Behandlung des Substrats oder der Imprägnierflüssigkeit, die zum Absorbieren von einer polaren Verbindung bestimmt ist, auf solche Weise, daß eine umgekehrte Polarität des Substrats und der Imprägnierflüssigkeit herbeigeführt wird, um ein hydrophiles/lipophiles Gleichgewicht (HLB) zu erzielen, so daß die Oberflächenspannung des Paares Träger — aktive Zusammensetzung niedriger ist als die Oberflächenspannung des Paares Träger — Wasser.

2. Verfahren nach Anspruch 1, dadurch gekennzeichnet, daß das Substrat ein poröses Absorptionsmittel ist.

3. Verfahren nach Anspruch 1 oder 2, dadurch gekennzeichnet, daß entsprechend den Anfangspolaritäten und den zu erzielenden Endpolaritäten die ionischen Zusatzverbindungen aus folgenden Klassen gewählt werden:

Verbindungen, die mindestens ein Heteroatom $P^+$, $O^+$, $S^+$ oder $N^+$ und mindestens eine lange Alkylkette mit mindestens 8 Kohlenstoffatomen enthalten, wie die quaternären Ammoniumsalze, die Imidazolinsalze, die Phosphoniumsalze, die Oxoniumsalze, die Fettamine und deren Derivate,

Verbindungen, die mindestens eine Gruppe mit anionischem Charakter, wie eine Carboxyl-, Sulfon-, Phosphor- und Schwefelgruppe, und mindestens eine lange Alkylkette mit mindestens 8 Kohlenstoffatomen enthalten, wie die Alkylphosphon-, Alkylschwefel-, Alkylsulfon- und Alkylcarbonsäuren,

mono- oder polyfunktionellen Verbindungen, wie organische Basen, Mineralbasen; Verbindungen mit mindestens bifunktionellem Character, die mindestens eine Gruppe mit negativem ionischen Charakter aufweisen und außerdem einen höheren positiven ionischen Charakter besitzen als jene der ionischen Gruppe des Trägers, wie monocarboxylische Polyamine, Peptide und Sulfobetaine;

bi- oder polyfunktionellen Verbindungen, die mindestens zwei positive ionische Gruppen aufweisen, wie die Polyamine und quaternären Polyammoniumverbindungen;

Verbindungen, die mindestens zwei negative ionische Gruppen aufweisen, wie die Polysäuren, mono- oder polyfunktionellen Verbindungen, wie die Mineralsäuren und die organischen Säuren,

Verbindungen mit mindestens bifunktionellem Charakter, die mindestens eine Gruppe mit positivem ionischen Charakter aufweisen und außerdem einen höheren negativen ionischen Charakter besitzen als jene der ionischen Gruppe des Trägers, wie die Monoaminpolysäuren, die Aminosäuren, die Peptide und die Sulfobetaine.

4. Verfahren nach einem der Ansprüche 1 bis 3, dadurch gekennzeichnet, daß die Menge der polaren Substanz ausreichend ist, um die beiden vereinigten Bedingungen (Polarität und HLB) zu erzielen, ohne die relativen Mengen der Bestandteile des Endsystems und ohne die wesentlichen Merkmale dieser Bestandteile und deren Wirkungsweise, insbesondere jene der chemisch aktiven Zusammensetzung, zu modifizieren.

5. Verfahren nach einem der Ansprüche 1 bis 3, dadurch gekennzeichnet, daß die Menge der polaren Substanz in bezug auf die aktive flüssige Zusammensetzung relativ niedrig ist.

6. Zusammensetzung, die die Desorption einer flüssigen chemisch aktiven, an ein Substrat gebundenen Zusammensetzung durch ein wässeriges Medium verhindert oder verzögert, dadurch gekennzeichnet, daß sie einen absorbierenden Träger, der mit der genannten aktiven Zusammensetzung imprägniert ist, und eine relativ kleine Menge mindestens einer ionischen polaren Verbindung umfaßt, wobei die Ladungen des Trägers und der chemisch aktiven Zusammensetzung entgegengesetzt sind und die Oberflächenspannung des Paares absorbierender Träger — chemisch aktive Substanz niedriger ist als die Oberflächenspannung des Paares absorbierender Träger — Wasser.

7. Zusammensetzung nach Anspruch 6, dadurch gekennzeichnet, daß die chemisch aktive Zusammensetzung ein Insektizid ist.

8. Anwendung des Verfahrens nach einem der Ansprüche 1 bis 5 zur Herstellung von Systemen, die gegen die Wirkung von Wasser, das mindestens eine chemisch aktive flüssige Zusammensetzung enthält, beständig sind.

9. Anwendung des Verfahrens nach einem der Ansprüche 1 bis 5 zur Herstellung von Umgebungsdesodorierungsmitteln für WC-Muschein und von Insektiziden.

# FIG.1

PARFUM A TYPE PIN

# FIG. 2

# FIG.3

PARFUM C   TYPE BOISE